(19) Europäisches Patentamt
European Patent Office
Office européen des brevets

(11) Veröffentlichungsnummer: **0 141 143**
**B1**

# (12) EUROPÄISCHE PATENTSCHRIFT

(45) Veröffentlichungstag der Patentschrift: **31.01.90**

(51) Int. Cl.⁴: **A 61 K 7/48**, C 07 C 33/20

(21) Anmeldenummer: **84110445.8**

(22) Anmeldetag: **03.09.84**

## (54) Sebosuppressive kosmetische Mittel, enthaltend Benzylalkohol-Derivate.

(30) Priorität: **09.09.83 DE 3332507**

(43) Veröffentlichungstag der Anmeldung: **15.05.85 Patentblatt 85/20**

(45) Bekanntmachung des Hinweises auf die Patenterteilung: **31.01.90 Patentblatt 90/5**

(84) Benannte Vertragsstaaten: **AT BE CH DE FR GB IT LI LU NL SE**

(56) Entgegenhaltungen:
**US-A- 2 850 410**
**US-A- 3 663 716**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Möller, Hinrich, Dr., Schumannstrasse 11, D-4019 Monheim (DE)**
Erfinder: **Wallat, Siegfried, Dr., Marie-Curie-Strasse 9, D-4019 Monheim (DE)**

**EP 0 141 143 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft topische, kosmetische Mittel zur Verbesserung des fettigen und unästhetischen Aussehens der Haare und der Haut, welche bestimmte Benzylalkohol-Derivate enthalten.

Die moderne Kosmetik ist bemüht, das durch übermäßig starke Absonderung der Talgdrüsen und der Kopfhaut verursachte fettige und wenig ästhetische Aussehen der Haare zu vermindern. Es ist daher vielfach versucht worden, durch geeignete Mittel die Sekretion der Talgdrüsen zu normalisieren, um dem Haar wieder sein gesundes Aussehen zu geben. Es wurden zur Bekämpfung der Soborrhoe des behaarten Kopfes kosmetische Pflegemittel mit Schwefel-, Quecksilber- oder Teerzusatz verwendet. Dabei hat sich gezeigt, daß diese bekannten antiseborrhoischen Zusätze bei längerer Anwendung häufig zu Nebenwirkungen führen, ohne daß wirklich befriedigende Ergebnisse im Hinblick auf Wirksamkeit und anwendungstechnische Eigenschaften erzielt werden konnten. In der DE-A-2 926 267 werden zur Normalisierung der Fettabsonderung 3,7,11-Trimethyl-2,6,10-dodecatrien-1-ol-Derivate als Zusatz zu kosmetischen Pflegemitteln beschrieben. Es hat sich jedoch gezeigt, daß diese Verbindungen nur eine sehr geringe antiseborrhoische Wirkung besitzen.

Aufgabe der Erfindung ist es, ein kosmetisches Mittel bereitzustellen, welches gegenüber den bekannten Präparaten eine verstärkte Wirkung ohne nachteilige Folgen auf den menschlichen Körper hat.

Es wurde nun überraschend gefunden, daß bestimmte Benzylalkohol-Derivate hervorragende antiseborrhoische Effekte auch bei sehr geringer Dosierung aufweisen.

Gegenstand der Erfindung sind sebosuppressive kosmetische Mittel, die gekennzeichnet sind durch einen Gehalt an Benzylalkohol-Derivaten oder allgemeinen Formel

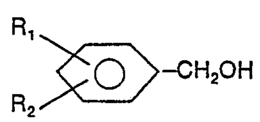

worin bedeuten

$R^1$ = Alkyl $C_4$–$C_{18}$, geradkettig oder verzweigt, oder Aryl, vorzugsweise Phenyl oder Alkyl-$C_4$–$C_{18}$-oxymethyl,

$R^2$ = H

oder

$R^1$ + $R^2$ = ankondensierter aromatischer Ring, vorzugsweise Benzolring,

wobei $R^1$ die 2-, 3- oder vorzugsweise 4-Stellung einnehmen kann.

Die anspruchsgemäß zu verwendenden Benzylalkohol-Derivate sind zum größten Teil bekannt und können nach allgemein bekannten Verfahren hergestellt werden.

Beispielsweise besteht ein Herstellungsverfahren in der Hydrolyse der entsprechenden Benzylhalogenide, wobei man vorteilhaft über die Stufe der Benzylacetate geht. Die Benzylhalogenide sind aus den entsprechend substituierten Aromaten durch Halogenmethylierung, insbesondere Chlormethylierung erhältlich. Ein anderes Verfahren beruht auf der Reduktion geeigneter aromatischer Carbonsäureester oder Aldehyde. Neben der katalytischen Hydrierung bietet sich die Reduktion mit komplexen Metallhydriden wie Natriumborhydrid, Lithiumaluminiumhydrid und Natrium-bis-(2-methoxyethoxy)-aluminiumdihydrid (Vitride®) an.

Als erfindungsgemäß einzusetzende Benzylalkohol-Derivate sind beispielsweise zu nennen: 4-Butyl-, 4-sek.butyl-, 4-tert.Butyl-, 4-Phenyl-, 4-Hexyl-, 4-Pentyl-, 4-Octyl-, 2-Octyl-, 4-Decyl-, 3-Decyl-, 4-Dodecyl-, 4-Undecyl-, 4-Tetradecyl-, 4-Hexadecyl-, 4-Octadecyl-, 4-(2-Ethylhexyl)-, 4-Isononyl-, 4-(2-Hexyldecyl)-, 4-Decyloxymethyl-, 4-Dodecyloxymethyl-benzylalkohol, sowie 1- und 2-Hydroxymethyl-naphthalin.

Die Verbindungen sind gut haut- und schleimhautverträglich und lassen sich ohne Schwierigkeiten in verschiedene kosmetische Zubereitungen, wie wäßrige oder alkoholische Lösungen, Öle, Suspensionen, Gele, Emulsionen, Salben oder Aerosole einarbeiten. Zur Behandlung von seborrhoischer Haut und fetten Haaren können diese in allen üblichen Applikationsformen, wie Haarwässern, Haarshampoos, Haarkuren, Haarspülungen, Hautlotionen oder Schüttelmixturen eingesetzt werden. Bevorzugt ist die Verwendung in Haarpflegemitteln. Neben der erfindungsgemäßen Wirkstoffkombination können diese kosmetischen Mittel übliche Träger- und Hilfsstoffe, wie Wasser, organische Lösungsmittel, oberflächenaktive Verbindungen, Öle, Fette, Wachse, Duftstoffe, Farbstoffe, Konservierungsmittel und dergleichen enthalten. Die neuen sebosuppressiven Mittel enthalten zweckmäßig 0,01–5,0 Gew.-%, vorzugsweise 0,05–1 Gew.-% der Benzylalkohol-Derivate.

Herstellungsbeispiele für Benzylalkohol-Derivate

A) 4-Octyl-benzylalkohol

Die Mischung aus 16,2 g (68 m Mol) 4-Octylbenzylchlorid, 41,8 g (0,43 Mol) Kaliumacetat und 200 ml Eisessig wurde 20 h auf 130 °C erwärmt, nach dem Abkühlen in ca. 1 l Wasser eingegossen und die wäßrige Phase mit tert. Butylmethylether extrahiert.

Die organische Phase wurde 2mal mit Wasser, mehrmals mit Kaliumhydrogencarbonat-Lösung (2 n) und anschließend mit gesättigter Natriumchlorid-Lösung gewaschen. Nach dem Abdestillieren des Lösungsmittels wurde der Rückstand in 160 ml 1,2-Dimethoxyethan aufgenommen und die Lösung nach Zusatz von 64 g Natriumhydroxid in 130 ml Wasser 45 h zum Sieden erhitzt. Nach Abdestillieren der Lösungsmittel wurde der Rückstand mit tert. Butylmethylether extrahiert, die Etherphase mit Wasser gewaschen, das Lösungsmittel abdestilliert, der Rückstand in Petrolether aufgenommen und die Lösung nach Behandeln mit Aktivkohle und Bleicherde erneut eingedampft.°

Nach Fraktionieren unter vermindertem Druck wurden 8 g (54% d. Th.) 4-Octyl-benzylalkohol vom Sdp. 128 C/0,11 m bar und dem Brechungsindex n : 1, 5033 erhalten.

B) 4-Decyl-benzylalkohol
wurde analog A) erhalten:
Schmp. 39–40 °C

C) 4-Dodecyl-benzylalkohol
wurde analog A) erhalten:
Schmp. 44–45 °C

d) 4-Decyloxymethyl-benzylalkohol

Zu 20 ml Diethylenglykoldimethylether wurden 0,25 g (6,7 m Mol) Natriumborhydrid und 0,58 g (6,7 m Mol) Lithiumbromid gegeben. Nach 0,5-stündigem Rühren wurden 3,3 g (10,8 m Mol) 4-Decyl-oxymethyl-benzoesäure-methylester (aus 4-Hydroxymethyl-benzoesäure-methylester und Decylbromid) hinzugefügt. Unter Rühren wurde die Mischung 3,5 h auf 100 °C erwärmt, abgekühlt und portionsweise auf Eis gegossen.

Nach Ansäuern der wäßrigen Phase mit 1,5 ml konz. Salzsäure wurde das Reaktionsprodukt mit Methylenchlorid extrahiert und nach dem Eindampfen der Methylenchlorid-Lösung säulenchromatographiert. (SiO/Methylenchlorid + 1% Methanol).

Nachdem zuerst noch unverändertes Ausgangsprodukt eluiert wurde, konnte aus den nachfolgenden Fraktionen 1 g 4-Decyclomethyl-benzylalkohol vom Schmp. 29–31 °C erhalten werden.

E) 4-Phenylbenzylalkohol und

F) 4-tert- Butylbenzylalkohol
sind im Handel erhältlich.

Die antiseborrhoische Wirkung wurde durch nachfolgend beschriebene Tierversuche näher untersucht.

Als Versuchstiere dienten männliche Wistar-Ratten von 220 bis 230 g Körpergewicht. Beurteilt wurde visuell der Bräunungsgrad auf dem Rücken der dort geschorenen Ratten. Die Bräunung wird durch das braune Hautoberflächenlipid der Ratten hervorgerufen. Dieser Test geht von der Beobachtung aus, daß junge weibliche Ratten sowie männliche Ratten nach dem Waschen mit Tensidlösung bzw. einem Lipidlösungsmittel und auch männliche Ratten, die systematisch mit Östrogen behandelt wurden, nur die normale heile, rosafarbene Haut nach dem Scheren aufweisen; parallel dazu sind aus den abgeschnittenen Haaren nur noch vergleichsweise sehr geringe Lipidmengen zu extrahieren.

Zur Beurteilung der Wirksamkeit wurden die Prüfsubstanzen in alkoholischer Lösung jeweils 6 Ratten halbseitig auf das Rückenfell gepinselt. Die andere Seite wurde nur mit dem Lösungsmittel ohne Wirkstoff behandelt.

Während der Versuchsdauer von 14 Tagen wurde an insgesamt 9 Tagen einmal appliziert. Zur weiteren Kontrolle diente eine Gruppe von 6 Ratten, die völlig unbehandelt blieben. Am Ende des Versuchs wurden die Tiere am Rücken und an den Flanken geschoren und von einem Beurteilungspanel (6 Personen) unabhängig unter Doppelblindbedingungen visuell abgemustert.

Bewertungsmethoden:

Als 1. Kriterium wurde bewertet, ob die Mehrheit der Beurteiler richtig die behandelte Seite erkannt hatten, wobei wie folgt differenziert wurde:

| Zeichen | Anteil der Beurteiler, die eine Wirkung erkannten |
|---|---|
| ++ | 100% |
| + | >50% – 100% |
| 0 | ≤50% |

Als 2. Kriterium wurde der Unterschied zwischen rechter und linker Seite gewertet, wobei pro Beurteiler und Tier jeweils 1 Punkt zu vergeben war, und zwar in der Weise, daß die

| | |
|---|---|
| dunklere Seite | mit 1 Punkt |
| hellere Seite | mit 0 Punkten und |
| bei Gleichheit beide Seiten | mit 0,5 Punkten |

benotet wurden.

Signifikante Differenzen zwischen unbehandelter und behandelter Seite nach der zweiten Bewertungsmethode zeigen die lokale Wirksamkeit einer Substanz an.

Als 3. Kriterium wurden außerdem noch die Intensitätsunterschiede der Brauntöne nach folgender Skala bewertet:

| | |
|---|---|
| 3 Punkte | stark braun |
| 2 Punkte | mittel braun |
| 1 Punkt | schwach braun |
| 0 Punkte | keine Braunfärbung. |

Nach der dritten Bewertungsmethode werden die Punktsummendifferenzen zwischen den unbehandelten Kontrolltieren und jeweils den behandelten und unbehandelten Seiten der Versuchstiere gebildet, wobei wiederum signifikante Differenzen zwischen Kontrolltieren und der behandelten Seite der Versuchstiere die Wirkung einer Substanz deutlich machen.

Parallel dazu ist in der Regel auch ein deutlicher Unterschied zwischen der unbehandelten und der behandelten Seite der Versuchstiergruppen zu sehen. Dieser ist aber nicht immer so deutlich wie der zwischen Kontrolltieren und behandelter Seite, wofür es verschiedene Gründe geben kann, wie zum Beispiel mechanische Substanzübertragung von einer auf die andere Seite oder Lösungsmitteleinfluß.

Zur Differenzierung der Effekte gemäß Beurteilungsmethode 2 und 3 wurde das folgende Schema verwendet:

| Zeichen | | Punktdifferenz |
|---|---|---|
| + + | sehr gross | (99,9% Wahrscheinlichkeit) |
| + | signifikant | (95 % Wahrscheinlichkeit) |
| 0 | minimal | (95 % Wahrscheinlichkeit) |

Prozentuale Sebumreduktion

Die Sebumreduktion errechnet sich aus der Punktedifferenz in der Weise, daß man den Quotienten aus der Punktedifferenz ΔP und der Punktezahl die Kontrollgruppe P bildet und den erhaltenen Wert in % angibt.

$$\text{Sebumreduktion} = \frac{P}{P_k} \cdot 100[\%]$$

Die Benzylalkohol-Derivate wurden in Konzentrationen von 0,5 bzw. 1,0% in Alkohol/Aceton (1:1) der angegebenen Weise appliziert. Die Ergebnisse sind in der Tabelle zusammengefaßt.

Bewertung der sebosuppressiven Effekte

| Substanz | Konz. (%ig) | Bewertungsmethode 1 | 2 | 3 | Sebumreduktion (%) |
|---|---|---|---|---|---|
| A | 0,5 | + + | + + | + + | 94 |
| B | 0,5 | + + | + + | + + | 95 |
| C | 0,5 | + + | + + | + + | 32 |
| D | 0,5 | + + | + + | + + | 82 |
| E | 0,5 | + + | + + | + + | 72 |
| F | 1,0 | + + | + + | + + | 56 |
| Benzylalkohol (US-A-3.663.716) | 1,0 | 0 | 0 | 0 | 0 |

Beispiele für kosmetische Rezepturen

Nachfolgend werden für die erfindungsgemäßen topischen Mittel zur Behandlung von stark fettendem Haar und seborrhoischer Haut Rezepturen gegeben:

1. Shampoo für fettendes Haar

| | Gewichtsteile |
|---|---|
| Ammoniumlaurylsulfat mit 33–35% Waschaktivsubstanz (Texapon a®) | 40,0 |
| Kokosfettsäurediethanolamid (Comperlan KD®) | 3,0 |
| Natriumchlorid | 2,0 |
| Natriumsulfat | 2,0 |
| 4-Decyl-benzylalkohol (B) | 0,5 |
| Konservierungsmittel | 0,1 |
| Parfümöl | 0,1 |
| Wasser | 52,3 |

2. Haarkur

| | |
|---|---|
| Glycerinmono-distearat (Tegin M®) | 0,7 |
| kationisches Tensid (z.B. Cetyltrimethylammoniumchlorid, Dehyquart A®) | 2,0 |
| Cholesterin | 2,0 |
| Sojalecithin | 0,2 |
| Emulgade A (Gemisch von Cetylalkohol mit nichtionogenen Emulgatoren) | 8,0 |
| Parfümöl | 0,3 |
| 4-Decyloxymethyl-benzylalkohol(D) | 0,2 |
| Wasser, vollentsalzt | 88,3 |

3. Hautcreme

| | |
|---|---|
| Selbstemulgierendes Gemisch aus Mono/Diglyceriden höherer gesättigter Fettsäuren mit Kaliumstearat (Cutina KD 16®) | 16,0 |
| Cetylstearylalkohol mit ca. 12 Mol Ethylenenoxid (Eumulgin B 1®) | 1,0 |
| 2-Octyldodecanol | 8,0 |
| Isopropylmyristat | 6,0 |
| Glycerin | 6,0 |
| 4-Octyl-benzylalkohol (A) | 0,2 |
| Wasser | 62,8 |

Bezugsquellen für die genannten Handelsprodukte:

| | |
|---|---|
| Texapon A® | = Henkel KGaA |
| Comperlan KD® | = Henkel KGaA |
| Cutina KD 16® | = Henkel KGaA |
| Eumulgin B 1® | = Henkel KGaA |
| Tegin M® | = Atlas Chemie |
| Dehyquart A® | = Henkel KGaA |

**Patentansprüche**

1. Sebosuppressive kosmetische Mittel, gekennzeichnet durch einen Gehalt an Benzylalkohol-Derivaten der allgemeinen Formel

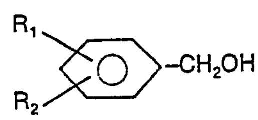

worin bedeuten

$R^1$ = Alkyl $C_4$–$C_{18}$ geradkettig oder verzweigt, oder Aryl, vorzugsweise Phenyl oder Alkyl-$C_4$–$C_{18}$-oxy-methyl,

$R^2$ = H

oder

$R^1 + R^2$ = ankondensierter aromatischer Ring, vorzugsweise Benzolring,

wobei $R^1$ die 2-, 3- oder vorzugsweise 4-Stellung einnehmen kann.

2. Mittel nach Anspruch 1, gekennzeichnet durch einen Gehalt von 0,01–5,0 Gew.-%, vorzugsweise 0,05–1,0 Gew.-% der Benzylalkohol-Derivate.

3. Verwendung von Mitteln nach einem der Ansprüche 1 oder 2 zur Herstellung von Arzneimitteln für die Behandlung von fettiger Haut und fettigen Haaren.

**Claims**

1. Sebosuppressive cosmetic preparations, characterized by a content of benzyl alcohol derivatives corresponding to the following general formula

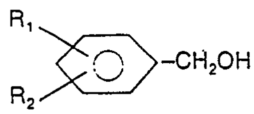

in which
$R^1$ represents a linear or branched $C_4$–$C_{18}$ alkyl radical or an aryl, preferably phenyl, radical or a $C_4$–$C_{18}$ alkyloxymethyl radical,
$R^2$ represents H or
$R^1$ and $R^2$ together form a fused aromatic ring, preferably a benzene ring,
$R^1$ occupying the 2-, 3- or, preferably, the 4-position.

2. Preparations as claimed in Claim 1, characterized by a content of from 0.01 to 5.0% by weight and preferably fromn 0.05 to 1.0% by weight of the benzyl alcohol derivatives.

3. The use of the preparations claimed in claim 1 or 2 for the production of medicaments for treating greasy skin and greasy hair.

**Revendications**

1. Agents cosmétiques sébosuppresseurs, caractérisés en ce qu'ils contiennent des dérivés d'alcool benzylique de formule générale:

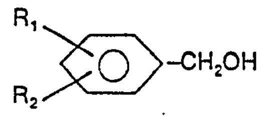

dans laquelle
$R^1$ représente un groupe alkyle en $C_4$–$C_{18}$, à chaîne droite ou ramifiée, ou un groupe aryle, de préférence, un groupe phényle, ou encore un groupe alkyl(en $C_4$–$C_{18}$)-oxyméthyle,
$R^2$ = H
ou
$R^1$ + $R^2$ = noyau aromatique condensé, de préférence, un noyau benzène,
$R^1$ pouvant occuper la position 2, la position 3 ou, de préférence, la position 4.

2. Agents selon la revendication 1, caractérisés en ce qu'ils contiennent 0,01–5,0% en poids, de préférence, 0,05–1,0% en poids des dérivés d'alcool benzylique.

3. Utilisation d'agents selon une des revendications 1 ou 2, pour la préparation de médicaments destinés au traitement de la peau grasse et des cheveux gras.